# EUROPEAN PATENT APPLICATION

(11) **EP 4 803 625 A1**
(43) Date of publication of application: **09.09.2026**
(21) Application number: 24883712.2
(22) Date of filing: 08.01.2024
(51) Int. Cl.: C12N 15/113, C12N 15/85, C12N 15/12, A01K 67/027

(54) **METHOD FOR CONSTRUCTING GENETICALLY-ENGINEERED ANIMAL USED FOR XENOTRANSFUSION**

(30) Priority: 03.11.2023 CN 202311467127
(71) Applicant: Clonorgan Biotechnology Co., Ltd., Chengdu, 610041 (CN)
(72) Inventor: DU, Jiaxiang, Chengdu Hi-Tech Zone (CN); WANG, Yong, Chengdu Hi-Tech Zone (CN); DENGKE, Pan, Chengdu Hi-Tech Zone (CN)
(74) Representative: Altmann Stößel Dick Patentanwälte PartG mbB
(86) International application number: PCT/CN2024/071051
(87) International publication number: WO 2025/091694

(57) **Abstract**

The present invention discloses a method for constructing genetically engineered animals suitable for xenogeneic blood transfusion, and belongs to the field of genetic engineering technology. The present invention provides a method for genetically modifying pig red blood cells (pRBCs), successfully achieves the expression of human protective proteins on pRBCs, enhances the compatibility of pRBCs in human immune system, reduces immune rejection, and lowers phagocytosis by macrophages. Relying on the fibroblast cells of α-gal, Sda, and Neu5Gc knockout pigs cultivated, the CRISPR-cas9 system is utilized to design efficient sgRNA, realizing site-specific integration of exogenous DNA sequences at the safe harbor site in pigs. The EF1α promoter is used to drive the expression of human protective proteins, enabling the expression of such proteins on the surface of pRBCs. The transgenic strategy provided in the present invention allows for stable expression of exogenous genes on the surface of pRBCs, further enhancing the survival ability of pRBCs in the human body. It can be used as a reserve of red blood cells from different species to alleviate the problem of insufficient blood reserves.

## Description

### Field of the invention

The present invention belongs to the field of genetic engineering technology, and specifically relates to a method for constructing genetically engineered animals suitable for xenogeneic blood transfusion.

### Background of the invention

With the rapid advancement of technology, the forms of local wars and the types of weapons in modern warfare have undergone earth-shattering changes. A large number of high-explosive and high-energy weapons have been put into use, significantly increasing lethality and destructiveness. The lethal area of weapons has gradually expanded, leading to a significant increase in the incidence of limb injuries. In the Gulf War, limb injuries accounted for over 80% of all war injuries. Severe penetrating injuries or blast injuries during wartime often cause fatal massive bleeding. Such patients are in critical and emergency conditions, and their treatment is limited by battlefield conditions. Due to insufficient supply of blood products, they may quickly develop hemorrhagic shock. Although limb replantation technology has been able to meet clinical needs, during the transportation process from the frontline battlefield, limbs are prone to ischemic necrosis, resulting in a high rate of disability. How to effectively treat these limb war-wounded patients, improve survival rates in battlefield and post-injury treatment, and reduce mortality and disability rates is an important challenge that needs to be urgently addressed in military medicine worldwide. The present invention meets the needs of modern warfare and explores the use of humanized gene-modified pig blood in the treatment of limb war wounds, aiming to improve the level of treatment for limb war wounds, reduce the amputation rate and mortality rate of limb war wounds, which is of great significance.

In addition, blood transfusion is one of the important measures to save lives, especially in cases of acute massive hemorrhage, severe anemia, and trauma surgery, where patients urgently need new blood to sustain their lives. In recent years, as the pandemic has ravaged the globe, there has been a severe shortage of blood sources, and blood products have been in short supply, with many blood banks in urgent need.

The act of transfusing non-human animal blood into humans is referred to as xenotransfusion. It has been found that porcine red blood cells (pRBCs) share very similar characteristics with human red blood cells (hRBCs), including cell size and lifespan, and pRBCs can survive normally in the human body. Humanized gene-modified red blood cells (RBCs) derived from pigs are not only convenient to obtain, but also facilitate the mass production of blood products, and can be directly stored in hospitals for future use.

However, there are three major obstacles in xenotransfusion: blood type incompatibility, immune rejection, and the risk of pathogenic microbial infection. The AO blood type of pigs is similar to the ABO blood type system of humans, and the blood type system compatibility can be ensured by screening for type O pigs. After pRBCs enter the human body, they undergo hemolysis due to immune rejection and agglutination reactions. Depending on the location of the hemolytic reaction, it can be divided into intravascular hemolysis and extravascular hemolysis. The former is mainly caused by the specific binding of pRBC surface heterologous antigens with natural antibodies in human serum, activating the complement system to form membrane-attacking complexes, leading to RBC lysis. The latter occurs when the Signal Regulatory Protein-α (SIPα) on the surface of human macrophages fails to recognize the "self" cell signal CD47 protein on the pRBC membrane, triggering phagocytosis by macrophages.

Transgenic pigs have been applied in many fields, yet they still face certain limitations, primarily due to the uncertainty of transgenic efficiency and integration sites. The CRISPR/Cas system, widely present in eubacteria and archaea, is an acquired immune system used to protect the host from secondary viral infection. This system functions by combining RNA transcribed from the CRISPR system with proteins translated from the Cas system to cleave DNA. The CRISPR/Cas system, which is currently the most widely used in the field of genome editing, is the Type II SpCas9 system. It utilizes a complex formed by a chimeric guide RNA, which is composed of crRNAs and tracrRNAs, and the nuclease Cas9 to recognize and cleave DNA, resulting in DNA double-strand breaks at specific loci in the genome. Compared to ZFNs and TALENs, the CRISPR/Cas9 system has rapidly become a mainstream technology in genome editing due to its advantages of extreme simplicity and low construction cost, and has become an indispensable research tool in modern life science laboratories. CD55 (Cluster of Differentiation 55), also known as Decay-accelerating Factor (DAF), is a complement regulatory protein encoded by CD55 gene. The molecular weight of human CD55 protein is 70 KDa, which can protect cells from the attack of the complement system by inhibiting the formation of the Membrane Attack Complex (MAC). Since mature RBCs lack a nucleus, current research reports have not yet achieved the expression of human complement regulatory protein CD55 on the surface of pRBCs.

Knocking out the major xenogeneic antigens in pigs can reduce the binding of antibodies in human serum by over 90%, but it still faces rejection mediated by complement and immune cells. Constructing transgenic pigs capable of expressing human complement regulatory proteins on the surface of RBCs holds significant importance for xenogeneic blood transfusion.

### Content of the invention

One objective of the present invention is to provide an sgRNA for constructing genetically engineered animals. Another objective of the present invention is to provide a method for constructing genetically engineered animals suitable for xenotransfusion, as well as the resulting genetically engineered animals and their uses.

The present invention provides a sgRNA, and its nucleotide sequence is set forth in SEQ ID NO:1, SEQ ID NO:2, SEQ ID NO:3, SEQ ID NO:4 or SEQ ID NO:5.

Further, the nucleotide sequence of said sgRNA is set forth in SEQ ID NO:3.

The present invention also provides another sgRNA, and its nucleotide sequence is set forth in SEQ ID NO:7, SEQ ID NO:8, SEQ ID NO:9 or SEQ ID NO:10.

Further, the nucleotide sequence of said sgRNA is set forth in SEQ ID NO:8.

The present invention also provides a method for constructing genetically engineered animals, which comprises the following steps:
(1) The sgRNA set forth in SEQ ID NO:1, SEQ ID NO:2, SEQ ID NO:3, SEQ ID NO:4, SEQ ID NO:5, SEQ ID NO:7, SEQ ID NO:8, SEQ ID NO:9 or SEQ ID NO:10 is connected with a vector to construct a knockout vector;
(2) Target DNA sequences expressing human protective proteins are constructed;
(3) Animal fibroblasts are co-transfected with knockout vectors and vectors containing target DNA sequences expressing human protective proteins, and positive clone cells with site-specific integration of human protective proteins are identified by sequencing;
(4) Using positive clone cells as nuclear donors and animal oocytes as recipients after enucleation, somatic cell nuclear transfer is performed to obtain reconstructed embryos;
(5) The reconstructed embryo is implanted into a female animal, the female animal is raised until it gives birth, and then the offspring is obtained.

Further, in step (1), the vector is a pX458 vector.

Further, in step (3), the vector containing the target DNA sequence expressing a human protective protein is a pUC57 cloning vector containing the target DNA sequence expressing a human protective protein.

In the present invention, "human protective protein" refers to a protein capable of enhancing the biocompatibility of heterologous cells within the host and improving the survival ability of these heterologous cells.

Further, in step (2), the human protective protein comprises one or both of human complement regulatory protein and human anti-cellular rejection protein; preferably, the human anti-cellular rejection protein is human anti-macrophage phagocytic protein or human anti-T cell activation protein; more preferably, the human complement regulatory protein is CD55, CD46, or CD59, the human anti-macrophage phagocytic protein is CD47, and the human anti-T cell activation protein is CTLA4.

Further, in step (3), the fibroblasts are fibroblasts with knockouts of heterologous antigens, and preferably fibroblasts with knockouts of α-gal, Sda, and Neu5Gc antigens.

Further, the animal is a pig.

Further, the method comprises the following steps:
(1) sgRNA is connected with the vector to construct knockout vector 1;
(2) Target DNA sequence 1 expressing human protective protein is constructed;
(3) Animal fibroblasts are co-transfected with knockout vector 1 and a vector containing the target DNA sequence 1 expressing human protective protein, and positive clone cells with site-specific integration of the human protective protein are identified by sequencing;
(4) Using the positive clone cells obtained in step (3) as nuclear donors, and the enucleated animal oocytes as recipients, somatic cell nuclear transfer is performed to obtain reconstructed embryos;
(5) The reconstructed embryo obtained in step (4) is implanted into a female animal, the female animal is raised until it gives birth, and then the transgenic animal is obtained by taking the offspring;
(6) sgRNA is connected with the vector to construct knockout vector 2;
(7) Target DNA sequence 2 expressing human protective protein is constructed;
(8) The knockout vector 2 and the vector containing the target DNA sequence 2 expressing human protective protein are co-transfected into the transgenic animal fibroblasts in step (5), and identify the positive clone cells with site-specific integration of human protective protein by sequencing;
(9) Using the positive clone cells obtained in step (8) as nuclear donors, and the enucleated animal oocytes as recipients, the somatic cell nuclear transfer is performed to obtain reconstructed embryos;
(10) The reconstructed embryo obtained in step (9) is implanted into a female animal, the female animal is raised until it gives birth, and then the offspring is obtained;
wherein, sgRNA mentioned in step (1) is set forth in SEQ ID NO:1, SEQ ID NO:2, SEQ ID NO:3, SEQ ID NO:4, or SEQ ID NO:5, the target DNA sequence 1 for expressing the human protective protein mentioned in step (2) is set forth in SEQ ID NO:6, sgRNA mentioned in step (6) is set forth in SEQ ID NO:7, SEQ ID NO:8, SEQ ID NO:9 or SEQ ID NO:10, and the target DNA sequence 2 for expressing the human protective protein mentioned in step (7) is set forth in SEQ ID NO:11; alternatively, sgRNA mentioned in step (1) is set forth in SEQ ID NO:7, SEQ ID NO:8, SEQ ID NO:9 or SEQ ID NO:10, the target DNA sequence 1 for expressing the human protective protein mentioned in step (2) is set forth in SEQ ID NO:11, sgRNA mentioned in step (6) is set forth in SEQ ID NO:1, SEQ ID NO:2, SEQ ID NO:3, SEQ ID NO:4, or SEQ ID NO:5, and the target DNA sequence 2 for expressing the human protective protein mentioned in step (7) is set forth in SEQ ID NO:6.

The present invention also provides a genetically engineered animal constructed by the above method.

The present invention also provides the use of genetically engineered animals mentioned above in xenogeneic blood transfusion.

Compared with the prior art, the present invention achieves the following beneficial effects: the present invention provides a method for genetically modifying pRBCs, successfully expressing human protective proteins on pRBCs, enhancing the compatibility of pRBCs in the human immune system, reducing immune rejection, and lowering phagocytosis by macrophages. Relying on the fibroblasts derived from Triple-Knockout (TKO) pigs cultivated, with knockout of antigens α-gal (GGTA1 gene knockout), Sda (B4GalNT2 gene knockout), and Neu5Gc (CMAH gene knockout), the CRISPR-cas9 system is used to design sgRNA, achieving site-specific integration of exogenous DNA sequences at a safe harbor site within the pig genome. The EF1α promoter is employed to drive the expression of human protective proteins, enabling the expression of such proteins on the surface of pRBCs. The transgenic strategy provided in the present invention enables stable expression of exogenous genes on the surface of pRBCs, further enhancing the survival ability of pRBCs in the human body. It can be used as a reserve of RBCs from heterologous sources to alleviate the problem of insufficient blood reserves.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Descriptions

Figure 1. Schematic diagram of target DNA sequence structure.
Figure 2. Identification of site-specific integration of CD55 transgene in TKO/CD55 transgenic pigs at the DNA level.
Figure 3. DNA sequencing results of TKO/CD55 transgenic pigs with knockouts of GGTA1, B4GalNT2, and CMAH genes.
Figure 4. Identification of expression of human CD55 protein on pRBCs in TKO/CD55 transgenic pigs using flow cytometry.
Figure 5. Comparison of agglutination effects of pRBCs from different genotypes of pigs.
Figure 6. Comparison of the binding effects of pRBCs from different genotypes of pigs with IgG and IgM antibodies in human serum.
Figure 7. CDC test results of pRBCs from different genotypes of pigs with human serum from various blood types.
Figure 8. Comparison of complement-mediated immune killing effects of pRBCs from different genotypes of pigs.
Figure 9. Identification results of TKO/CD55/CD47 transgenic pigs at DNA levels.
Figure 10. Identification of expression of human CD47 protein on pRBCs in TKO/CD55/ CD47 transgenic pigs using flow cytometry.
Figure 11. The results of phagocytosis of pRBCs from different genotypes of pigs by monkey macrophages.

### Examples

The raw materials and equipment used in the present invention are all known products, obtained by purchasing those commercially available.

The triple-knockout (TKO) pigs with knockout of antigens α-gal (GGTA1 gene knockout), Sda (B4GalNT2 gene knockout), and Neu5Gc (CMAH gene knockout) are sourced from Chengdu Zhongke Aoge Biotechnology Co., Ltd.

### Example 1: Construction method of GGTA1KO/B4GALNT2KO/CMAHKO/hCD55 transgenic pigs (referred to as TKO/CD55 transgenic pigs)

Step 1: sgRNA specifically recognizing the target sequence DNA was designed at the ROSA26 locus in the pig genome, and ligated into the BbsI-digested and recovered pX458 vector to construct a knockout vector. The knockout efficiency was verified, and efficient sgRNA (Table 1) was selected for subsequent experiments.

**Table 1. sgRNA sequences and their knockout efficiency.**

| Sequence No. | sgRNA name | sgRNA sequence | Knockout efficiency |
|---|---|---|---|
| SEQ ID NO:1 | Rosa26-sgRNA-1 | gaaggccgcacccttctccgc | 54.17% (13/24) |
| SEQ ID NO:2 | Rosa26-sgRNA-2 | gtctgctgcctccttttccta | 39.13% (9/23) |
| SEQ ID NO:3 | Rosa26-sgRNA-3 | ggctccttctcgattatgggc | 86.36% (19/22) |
| SEQ ID NO:4 | Rosa26-sgRNA-4 | gccctggcttaacctgattct | 52.38% (11/21) |
| SEQ ID NO:5 | Rosa26-sgRNA-5 | gattcttgggcgttgtcctgc | 69.57% (16/23) |

The verification method for knockout efficiency was as follows:
The sgRNA gene knockout plasmid was transfected into the fibroblast cell lines of porcine ears, and after 48-72 hours of culture following transfection, the GFP-positive cell population was sorted and recovered by flow cytometry. DNA was extracted from the positive cell population, the knockout target sequence was amplified by PCR, the target sequence was recovered by gel purification, and cloned into T vector using TA cloning. 20-30 monoclonal colonies were picked for sequencing.

The verification results are presented in Table 1. It was observed that pX458-Rosa26-sgRNA-3 exhibited the highest knockout efficiency, and thus, pX458-Rosa26-sgRNA-3 was selected for subsequent experiments.

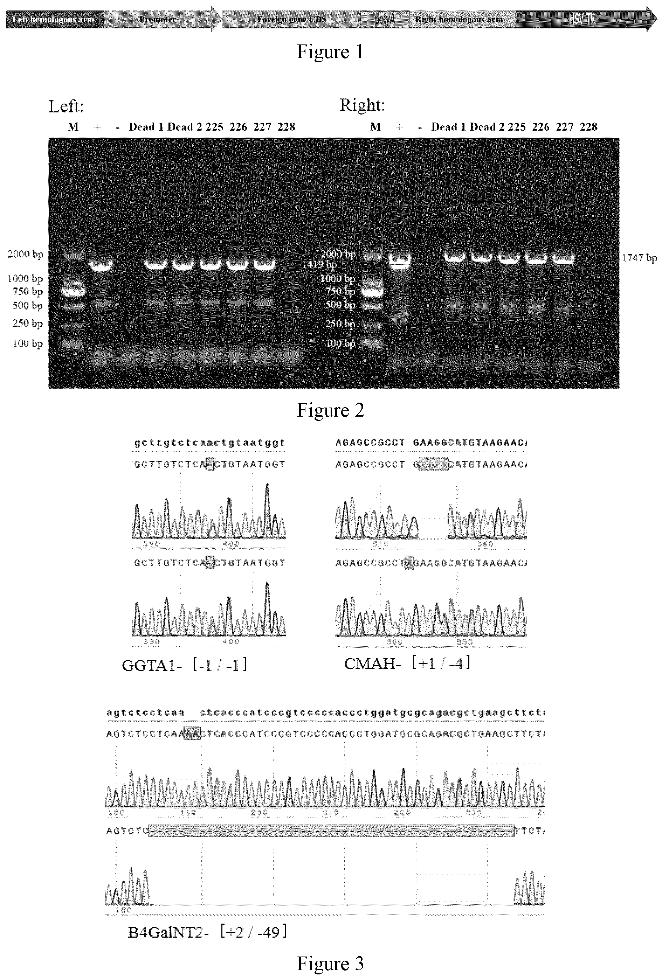

Step 2: The left homologous arm, EF1α promoter, CDS sequence of human CD55 gene, polyA sequence, right homologous arm, and HSV-TK sequence (including promoter and polyA) were sequentially ligated by homologous recombination technology. The left and right ends are respectively added with the sequences of restriction endonuclease sites NheI and HindIII. The target DNA sequence was ligated to the pUC57 cloning vector. Figure 1 is the schematic diagram of the target DNA sequence structure.

The target DNA sequence is:

GTCACTGATTGGCCGCTTCTCCTCCCGCCGTGTGTGAAAACACAAATGGCGTG
TTTTGGTTGGAGTAAAGCTCCTGTCAGTTACAGCCTCGGGAGTGCGCAGCCTCCCA
GGAACTCTCGCATTGCCCCCTGGGTGGGTAGGTAGGTGGGGTGGAGAGAGCTGCA
CAGGCGGGCGCTGTCGGCCTCCTGCGGGGGGAGGGGAGGGTCAGTGAAAGTGGCT
CCCGCGCGGGCGTCCTGCCACCCTCCCCTCCGGGGGAGTCGGTTTACCCGCCGCCT
GCTCGGCTTTGGTATCTGATTGGCTGCTGAAGTCCTGGGAACGGCCCCTTGTTATTG
GCTTGGGTCCCAAATGAGCGAAACCACTACGCGAGTCGGCAGGGAGGCGGTCTTT

GGTACGGCCCTCCCCGAGGCCAGCGCCGCAGTGTCTGGCCCCTCGCCCCTGCGCAA
CGTGGCAGGAAGCGCGCGCAGGAGGCGGGGGCGGGCTGCCGGGCCGAGGCTTCTG
GGTGGTGGTGACTGCGGCTCCGCCCTGGGCGTCCGCCGCCTGAAGGACGAGACTAG
CTCTCTACCTGCTCTCGGACCCGTGGGGGTGGGGGGTGGAGGAAGGAGTGGGGGG
TCGGTCCTGCTGGCTTGTGGGTGGGAGGCGCATGTTCTCCAAAAACCCGCGCGAGC
TGCAATCCTGAGGGAGCTGCAGTGGAGGAGGCGGAGAGAAGGCCGCACCCTTCTC
CGCAGGGGGAGGGGAGTGCCGCAATACCTTTATGGGAGTTCTCTGCTGCCTCCTTT
TCCTAAGGACCGCCCTGGGCCTAGAAAAATCCCTCCCTCCCCCGCGATCTCGTCAT
CGCCTCCATGTCAGTTTGGCTCCGGTGCCCGTCAGTGGGCAGAGCGCACATCGCCC
ACAGTCCCCGAGAAGTTGGGGGGAGGGGTCGGCAATTGAACCGGTGCCTAGAGAA
GGTGGCGCGGGGTAAACTGGGAAAGTGATGTCGTGTACTGGCTCCGCCTTTTTCCC
GAGGGTGGGGGAGAACCGTATATAAGTGCAGTAGTCGCCGTGAACGTTCTTTTTCG
CAACGGGTTTGCCGCCAGAACACAGGTAAGTGCCGTGTGTGGTTCCCGCGGGCCTG
GCCTCTTTACGGGTTATGGCCCTTGCGTGCCTTGAATTACTTCCACGCCCCTGGCTG
CAGTACGTGATTCTTGATCCCGAGCTTCGGGTTGGAAGTGGGTGGGAGAGTTCGAG
GCCTTGCGCTTAAGGAGCCCCTTCGCCTCGTGCTTGAGTTGAGGCCTGGCTTGGGC
GCTGGGGCCGCCGCGTGCGAATCTGGTGGCACCTTCGCGCCTGTCTCGCTGCTTTCG
ATAAGTCTCTAGCCATTTAAAATTTTTGATGACCTGCTGCGACGCTTTTTTTCTGGC
AAGATAGTCTTGTAAATGCGGGCCAAGATCTGCACACTGGTATTTCGGTTTTTGGG
GCCGCGGGCGGCGACGGGGCCCGTGCGTCCCAGCGCACATGTTCGGCGAGGCGGG
GCCTGCGAGCGCGGCCACCGAGAATCGGACGGGGGTAGTCTCAAGCTGGCCGGCC
TGCTCTGGTGCCTGGCCTCGCGCCGCCGTGTATCGCCCCGCCCTGGGCGGCAAGGC
TGGCCCGGTCGGCACCAGTTGCGTGAGCGGAAAGATGGCCGCTTCCCGGCCCTGCT
GCAGGGAGCTCAAAATGGAGGACGCGGCGCTCGGGAGAGCGGGCGGGTGAGTCAC
CCACACAAAGGAAAAGGGCCTTTCCGTCCTCAGCCGTCGCTTCATGTGACTCCACG
GAGTACCGGGCGCCGTCCAGGCACCTCGATTAGTTCTCGAGCTTTTGGAGTACGTC
GTCTTTAGGTTGGGGGGAGGGGTTTTATGCGATGGAGTTTCCCCACACTGAGTGGG
TGGAGACTGAAGTTAGGCCAGCTTGGCACTTGATGTAATTCTCCTTGGAATTTGCCC
TTTTTGAGTTTGGATCTTGGTTCATTCTCAAGCCTCAGACAGTGGTTCAAAGTTTTT
TTCTTCCATTTCAGGTGTCGTGAGGATCTATTTCCGGTGAATTCCTCGAGACTAGTTC
TAGAGCCCACCATGACCGTCGCGCGGCCGAGCGTGCCCGCGGCGCTGCCCCTCCTC
GGGGAGCTGCCCCGGCTGCTGCTGCTGGTGCTGTTGTGCCTGCCGGCCGTGTGGGG
TGACTGTGGCCTTCCCCCAGATGTACCTAATGCCCAGCCAGCTTTGGAAGGCCGTA
CAAGTTTTCCCGAGGATACTGTAATAACGTACAAATGTGAAGAAAGCTTTGTGAAA

ATTCCTGGCGAGAAGGACTCAGTGATCTGCCTTAAGGGCAGTCAATGGTCAGATAT
TGAAGAGTTCTGCAATCGTAGCTGCGAGGTGCCAACAAGGCTAAATTCTGCATCCC
TCAAACAGCCTTATATCACTCAGAATTATTTTCCAGTCGGTACTGTTGTGGAATATG
AGTGCCGTCCAGGTTACAGAAGAGAACCTTCTCTATCACCAAAACTAACTTGCCTT
CAGAATTTAAAATGGTCCACAGCAGTCGAATTTTGTAAAAAGAAATCATGCCCTAA
TCCGGGAGAAATACGAAATGGTCAGATTGATGTACCAGGTGGCATATTATTTGGTG
CAACCATCTCCTTCTCATGTAACACAGGGTACAAATTATTTGGCTCGACTTCTAGTT
TTTGTCTTATTTCAGGCAGCTCTGTCCAGTGGAGTGACCCGTTGCCAGAGTGCAGA
GAAATTTATTGTCCAGCACCACCACAAATTGACAATGGAATAATTCAAGGGGAACG
TGACCATTATGGATATAGACAGTCTGTAACGTATGCATGTAATAAAGGATTCACCA
TGATTGGAGAGCACTCTATTTATTGTACTGTGAATAATGATGAAGGAGAGTGGAGT
GGCCCACCACCTGAATGCAGAGGAAAATCTCTAACTTCCAAGGTCCCACCAACAGT
TCAGAAACCTACCACAGTAAATGTTCCAACTACAGAAGTCTCACCAACTTCTCAGA
AAACCACCACAAAAACCACCACACCAAATGCTCAAGCAACACGGAGTACACCTGT
TTCCAGGACAACCAAGCATTTTCATGAAACAACCCCAAATAAAGGAAGTGGAACC
ACTTCAGGTACTACCCGTCTTCTATCTGGGCACACGTGTTTCACGTTGACAGGTTTG
CTTGGGACGCTAGTAACCATGGGCTTGCTGACTTAGGCCTCGACTGTGCCTTCTAGT
TGCCAGCCATCTGTTGTTTGCCCCTCCCCCGTGCCTTCCTTGACCCTGGAAGGTGCC
ACTCCCACTGTCCTTTCCTAATAAAATGAGGAAATTGCATCGCATTGTCTGAGTAG
GTGTCATTCTATTCTGGGGGGTGGGGTGGGGCAGGACAGCAAGGGGGAGGATTGG
GAAGACAATAGCAGGCATGCTGGGGCTAGCGCGGCCGCGGATAACTTCGTATAGC
ATACATTATACGAAGTTATGGGATTCTTTTGCCCTGGCTTAACCTGATTCTTGGGCG
TTGTCCTGCAGGGGATTGAGCAGGTGTACGAGGACGAGCCCAATTTCTCTATATTC
CCACAGTCTTGAGTTTGTGTCACAAAATAATTATAGTGGGGTGGAGATGGGAAATG
AGTCCAGGCAACACCTAAGCCTGATTTTATGCATTGAGACTGCGTGTTATTACTAA
AGATCTTTGTGTCGCAATTTCCTGATGAAGGGAGATAGGTTAAAAAGCACGGATCT
ACTGAGTTTTACAGTCATCCCATTTGTAGACTTTTGCTACACCACCAAAGTATAGCA
TCTGAGATTAAATATTAATCTCCAAACCTTAGGCCCCCTCACTTGCATCCTTACGGT
CAGATAACTCTCACTCATACTTTAAGCCCATTTTGTTTGTTGTACTTGCTCATCCAGT
CCCAGTCCCATTGGCTTTCTCCTCACCTGTTTTAGGTAGCCAGCAAGTCATGAAATC
AGATAAGTTCCACCACCAATTAACACTACCCATCTTGAGCATAGGCCCAACAGTGC
ATTTATTCCTCATTTACTGATGTTCGTGAATATTTACCTTGATTTTCATTTTTTTCTTT
TTCTTAAGCTGGGATTTTACTCCTGACCCTATTCACAGTCAGATGATCTTGACTACC
ACTGCGATTGGACCTGAGGTTCAGCAATACTCCCCTTTATGTCTTTTGAATACTTTT

CAATAAATCTGTTTGTATTTTCATTAGTTAGTAACTGAGCTCAGTTGCCGTAATGCT
AATAGCTTCCAAACTAGTGTCTCTGTCTCCAGTATCTGATAAATCTTAGGTGTTGCT
GGGACAGTTGTCCTAAAATTAAGATAAAGCATGAAAATAACTGACACAACTCCATT
ACTGGCTCCTAACTACTTAAACAATGCATTCTATCATCACAAATGTGAAAAAGGAG
TTCCCTCAGTGGACTAACCTTATCTTTTCTCAACACCTTTTTCTTTGCACAATTTTCC
ACACATGCCTACAAAAAGTACTTCTCTGCTCAAGTCACACTGAGTTGATTGCTATTT
ACCGAAATCAAAGTAACATTATCAGATCTCTGTAGGGTGGTTCCCTCTGGAATGCT
ACCCTCCATAGTCCTTACCCTTCAAGTAAAGAGCATGAAGACTGAAATATCTCTGT
GATCTGTCATCCTTTAAGCCAGAATCCCCCATAAAAAAGTTAGTATTGCTTTCTCCT
GATCCCATAGCAGGTTGAATCATAGCACTTATCAGGTTGTTGTCATTGCTTGCTTAA
ATTCTCCTAACTATTTGGAGCTTCTTGAGGGCACAGGTTCTTGTTGAGTCTTGTACC
TAAGCACCTAGTATAGTCCTTGATGTCTAGCCAACCCTAAATAAAATGCAGTGAGT
GACATGTAGATGTCTTTATAAGGTTTGATAGGTTGGTCTCTCAAACAGTTCTTTTGT
ATGTTTGGTAGTGCTCTAGATTAGCACTGGCCAGTATAACTCTGATGATGGAAATG
TTCTATAGCTATGCTGTCTAATATGGTA (SEQ ID NO:6).

Step 3: The constructed cloning vector was transfected into competent cells, and positive bacteria were screened and propagated. The plasmid was extracted and subjected to double enzyme digestion using NheI and HindIII restriction endonucleases. The target DNA sequence was recovered by gel purification, and then it and the Rosa26-sgRNA-3 knockout vector were co-transfected into fibroblasts of pigs with knockout of antigens α-gal, Sda, and Neu5Gc (Table 2). GFP-positive cell populations were recovered by flow cytometry, and monoclonal cells were screened by limiting dilution. After culturing for about 10 days, monoclonal cells were picked up for DNA level identification, and identified using PCR technology to obtain positive clones with CD55 site-specific integration.

Step 4: By somatic cell cloning technology, the cells with the positive clones were injected into the enucleated oocytes for fusion, and then the reconstructed embryo was transferred into the uterus of the recipient sow for pregnancy.

Step 5: The recipient sows gave birth naturally to 4 healthy piglets and 2 weak piglets (which died), resulting in the construction of TKO/CD55 transgenic pigs.

The following steps involved DNA level identification, protein level identification, and protection effect verification for the born piglets:

### (1) Identification at DNA level

After the piglet was born, a small amount of ear tissue was cut, and then DNA was extracted from the piglet's ear sample using a DNA extraction kit according to the instructions. After DNA extraction, primers were designed based on the target CD55 transgenic DNA sequence for identification. There are two pairs of primers, one pair near each of the left and right homologous arms, to identify the site-specific integration of CD55. After PCR amplification, the products were subjected to gel electrophoresis to identify the site-specific integration of the left and right arms. All piglets exhibited site-specific integration of CD55 (Figure 2). Simultaneously, PCR amplification was performed on the GGTA1, B4GALNT2, and CMAH gene editing target sequences. The target sequences were recovered using a gel extraction kit and cloned into T vector. Ten monoclonal colonies were picked for sequencing identification. By TA cloning and sequencing, it was confirmed that the GGTA1, B4GALNT2, and CMAH genes in all piglets had been knocked out (Figure 3).

### (2) Identification at protein level

When piglets reached 1 month of age, 5 mL of whole pig blood was collected, centrifuged at 900 g for 5 min, and then washed twice with PBS. After separation of RBCs, 10⁶ RBCs were taken out, to which was added 5 µL of PE-labeled CD55 mouse anti-human antibody, and incubated at 4 °C for 30 min, followed by washing three times with PBS. The cells were re-suspended, and the expression level of exogenous proteins on the surface of RBCs was identified by flow cytometry. The expression level of CD55 was represented by the average fluorescence intensity. Wild-type pRBCs and hRBCs were used as controls.

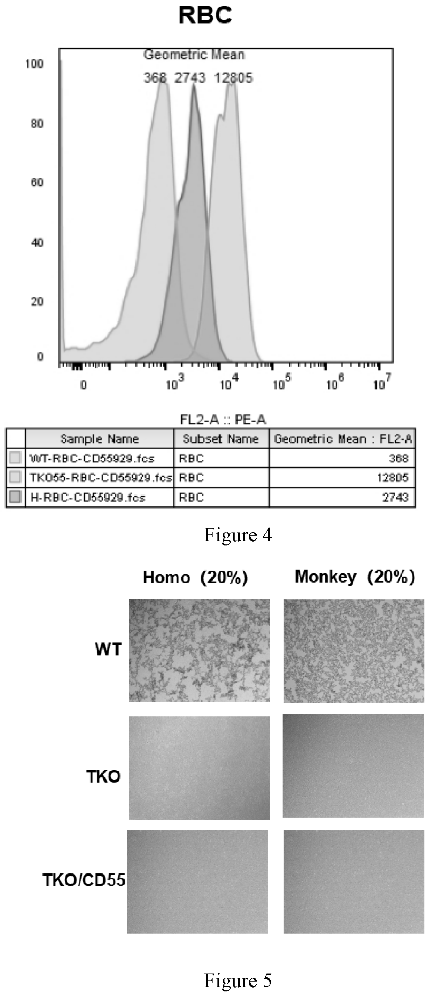

The results are shown in Figure 4. The RBCs from wild-type pigs did not express human CD55 (orange), while human CD55 expression could be detected on the surface of RBCs in TKO/CD55 transgenic pigs. Compared with hRBCs (red), the expression level of human CD55 on RBCs of TKO/CD55 transgenic pigs (blue) was approximately four times higher.

### (3) Verification of protective effect

When piglets reached 1 month of age, 5 mL of whole pig blood was collected, centrifuged at 900 g for 5 min, and washed twice with PBS. Then, the RBCs were separated. The protective effect was verified by *in vitro* RBC agglutination test, IgG/IgM antibody binding detection, and CDC (complement dependent cytotoxicity) experiment. Using wild-type pigs (WT) as well as a-gal, Sda, and Neu5Gc knockout pigs (Triple-Knockout; TKO) as controls, Homo type A represents the inactivated serum of individuals with A blood type, Homo type B represents the inactivated serum of individuals with B blood type, Homo type AB represents the inactivated serum of individuals with AB blood type, and Homo type O represents the inactivated serum of individuals with O blood type.

The method for the *in vitro* RBC agglutination test was as follows: 10⁶ RBCs were taken and incubated with 25% heat-inactivated mixed human serum at room temperature for 30 min. Then, the agglutination of RBCs was observed under a microscope.

The IgG/IgM antibody binding assay was as follows: 10⁶ RBCs were taken and incubated with 25% heat-inactivated mixed human serum at 4°C for 30 min, followed by washing twice with PBS, to which were then added fluorescently labeled anti-human IgG antibody and fluorescently labeled anti-human IgM antibody, respectively. The cells were incubated at 4 °C for 30 min, and washed twice with PBS. The cells were re-suspended in PBS. The fluorescence intensity on the surface of RBCs was detected using flow cytometry.

The CDC experimental method was as follows: 10⁶ RBCs were taken out and incubated with 25% heat-inactivated human serum at 4 °C for 30 min, followed by washing once with PBS, to which was added 25% rabbit complement. The cells were incubated at 37 °C for 30 min, and then centrifuged at 900 g for 5 min. 100 µL of the supernatant was transferred to a 96-well plate. The OD560 value of the sample was measured using a microplate reader, to calculate the mortality rate of RBCs. The mortality rate of RBCs = ([A - C]/[B - C]) × 100%, wherein A represents the experimental value (OD), B represents the maximum mortality group (RBCs lysed by lysis buffer), and C represents the minimum mortality group (the negative control, without addition of complement and serum). Each experiment was repeated three times.

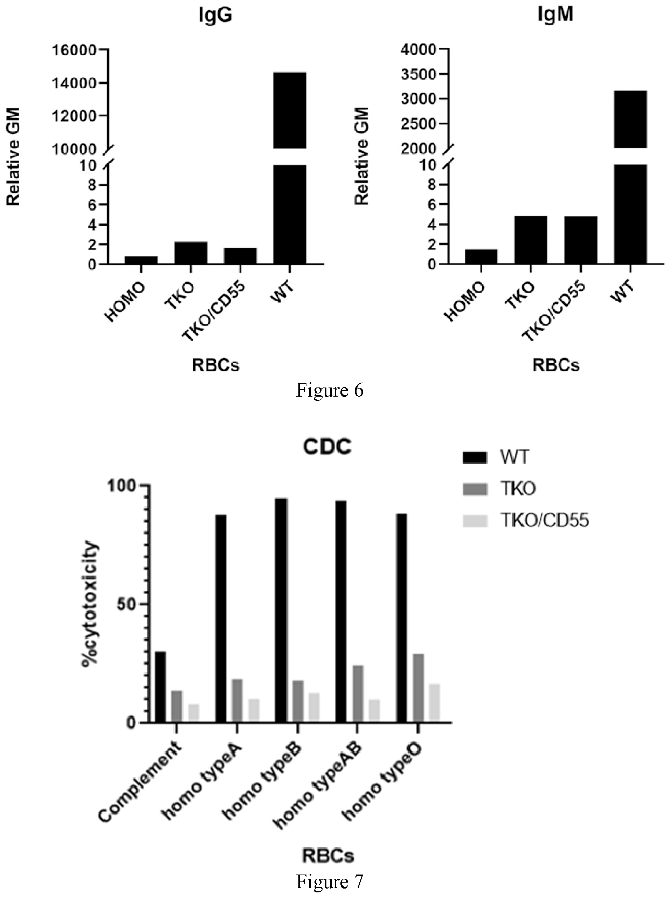

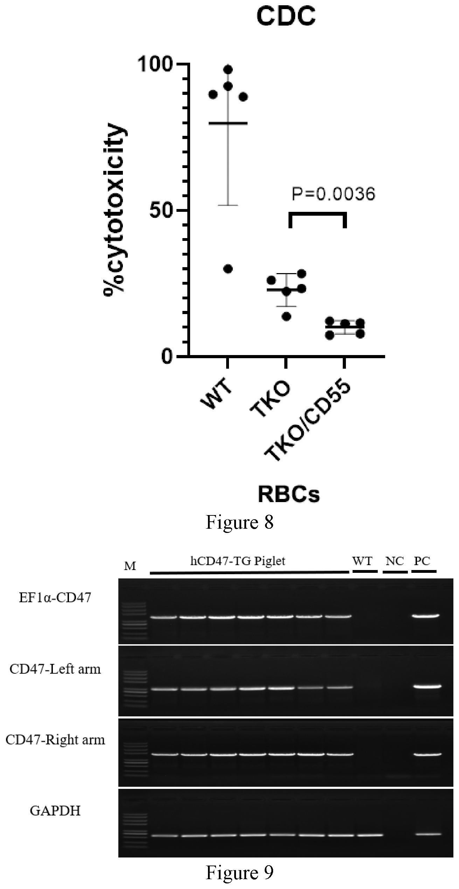

The results showed that:
1. Compared with wild-type pigs, removing three antigens from pigs could reduce the degree of agglutination (Figure 5);
2. The removal of three pig antigens significantly reduced the binding of pRBCs to IgG and IgM antibodies in human serum. The antibody binding levels of RBCs in TKO and TKO/CD55 pigs were essentially consistent, with no significant difference (Figure 6);
3. CDC tests were conducted on pRBCs and human sera of different blood types, and there was no significant difference in the results for different blood types and the RBCs from the same genotype of pigs (Figure 7);
4. CDC tests were conducted on the RBCs separately from WT, TKO/CD55, and TKO pigs and mixed human serum. The results showed that the removal of three pig antigens significantly inhibited complement-mediated immune killing. The introduction of human CD55 gene further suppressed the complement-mediated killing effect (Figure 8).

### Example 2: Construction method of GGTA1KO/B4GALNT2KO/CMAHKO/hCD55/hCD47 transgenic pigs (referred to as TKO/CD55/CD47 transgenic pigs)

After successfully obtaining TKO/CD55 transgenic pigs in Example 1, it was verified that the EF1α promoter could be used to express exogenous proteins on RBCs. This successfully achieved the expression of the human protective protein CD55 on pRBCs, enhancing the compatibility of pRBCs in the human immune system and reducing immune rejection.

Based on the above, in this example, the human anti-macrophage phagocytic protein CD47 was further transferred into the H11 locus of TKO/CD55 transgenic pigs, to reduce the phagocytosis of pRBCs by macrophages. The specific steps were as follows:
Step 1: By reference to the method in Example 1, sgRNA that specifically recognizes the target sequence DNA was designed at the H11 locus of the pig genome, and ligated into the BbsI enzyme-cleaved and recovered pX458 vector to construct a knockout vector. The knockout efficiency was verified, and efficient sgRNA (Table 2) was selected for subsequent experiments.

**Table 2. sgRNA sequence and the knockout efficiency**

| Sequence No. | sgRNA name | sgRNA sequence | Knockout efficiency |
|---|---|---|---|
| SEQ ID NO:7 | H11-sgRNA-1 | GAGGCCATTCTCTGATGGAC | 50% (15/30) |
| SEQ ID NO:8 | H11-sgRNA-2 | GTGACTATATTTTGACTAAA | 92% (26/28) |
| SEQ ID NO:9 | H11-sgRNA-3 | GAGCATGGTCTGTCTCTTCC | 76% (19/25) |
| SEQ ID NO:10 | H11-sgRNA-4 | GTACTTCATTAGTCCCCTAAG | 52% (12/23) |

The verification results are presented in Table 2. It was observed that H11-sgRNA-2 exhibited the highest knockout efficiency, and thus, H11-sgRNA-2 was selected for subsequent experiments.

Step 2: Following the method described in Example 1, an integration vector was constructed for the human CD47 gene using homologous recombination technology.

The target DNA sequence was:

AGCTTGGCGTAATCATGGTCATAGCTGTTTCCTGTGTGAAATTGTTATCCGCTCA
CAATTCCACACAACATACGAGCCGGAAGCATAAAGTGTAAAGCCTGGGGTGCCTAA
TGAGTGAGCTAACTCACATTAATTGCGTTGCGCTCACTGCCCGCTTTCCAGTCGGGA
AACCTGTCGTGCCAGCTGCATTAATGAATCGGCCAACGCGCGGGGAGAGGCGGTTT
GCGTATTGGGCGCTCTTCCGCTTCCTCGCTCACTGACTCGCTGCGCTCGGTCGTTCG
GCTGCGGCGAGCGGTATCAGCTCACTCAAAGGCGGTAATACGGTTATCCACAGAATC
AGGGGATAACGCAGGAAAGAACATGTGAGCAAAAGGCCAGCAAAAGGCCAGGAA

CCGTAAAAAGGCCGCGTTGCTGGCGTTTTTCCATAGGCTCCGCCCCCCTGACGAGCA
TCACAAAAATCGACGCTCAAGTCAGAGGTGGCGAAACCCGACAGGACTATAAAGAT
ACCAGGCGTTTCCCCCTGGAAGCTCCCTCGTGCGCTCTCCTGTTCCGACCCTGCCGC
TTACCGGATACCTGTCCGCCTTTCTCCCTTCGGGAAGCGTGGCGCTTTCTCATAGCTC
ACGCTGTAGGTATCTCAGTTCGGTGTAGGTCGTTCGCTCCAAGCTGGGCTGTGTGCA
CGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTA
GCAGAGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTACCTTC
GGAAAAAGAGTTGGTAGCTCTTGATCCGGCAAACAAACCACCGCTGGTAGCGGTGG
TTTTTTTGTTTGCAAGCAGCAGATTACGCGCAGAAAAAAAGGATCTCAAGAAGATC
CTTTGATCTTTTCTACGGGGTCTGACGCTCAGTGGAACGAAAACTCACGTTAAGGGA
TTTTGGTCATGAGATTATCAAAAAGGATCTTCACCTAGATCCTTTTAAATTAAAAATG
AAGTTTTAAATCAATCTAAAGTATATATGAGTAAACTTGGTCTGACAGTTACCAATGC
TTAATCAGTGAGGCACCTATCTCAGCGATCTGTCTATTTCGTTCATCCATAGTTGCCTG
ACTCCCCGTCGTGTAGATAACTACGATACGGGAGGGCTTACCATCTGGCCCCAGTGC
TGCAATGATACCGCGAGACCCACGCTCACCGGCTCCAGATTTATCAGCAATAAACCA
GCCAGCCGGAAGGGCCGAGCGCAGAAGTGGTCCTGCAACTTTATCCGCCTCCATCC
AGTCTATTAATTGTTGCCGGGAAGCTAGAGTAAGTAGTTCGCCAGTTAATAGTTTGCG
CAACGTTGTTGCCATTGCTACAGGCATCGTGGTGTCACGCTCGTCGTTTGGTATGGC
TTCATTCAGCTCCGGTTCCCAACGATCAAGGCGAGTTACATGATCCCCCATGTTGTGC
AAAAAAGCGGTTAGCTCCTTCGGTCCTCCGATCGTTGTCAGAAGTAAGTTGGCCGC
AGTGTTATCACTCATGGTTATGGCAGCACTGCATAATTCTCTTACTGTCATGCCATCCG
TAAGATGCTTTTCTGTGACTGGTGAGTACTCAACCAAGTCATTCTGAGAATAGTGTAT
GCGGCGACCGAGTTGCTCTTGCCCGGCGTCAATACGGGATAATACCGCGCCACATAG
CAGAACTTTAAAAGTGCTCATCATTGGAAAACGTTCTTCGGGGCGAAAACTCTCAA
GGATCTTACCGCTGTTGAGATCCAGTTCGATGTAACCCACTCGTGCACCCAACTGAT
CTTCAGCATCTTTTACTTTCACCAGCGTTTCTGGGTGAGCAAAAACAGGAAGGCAA
AATGCCGCAAAAAAGGGAATAAGGGCGACACGGAAATGTTGAATACTCATACTCTT
CCTCCCAAGCTTAGCTATGGCCGTTTCTTGAGCCTTAATAAAGACCCCAATGGGATAG
TTCAGGCTGGGCTGACCCTATAGGTAGGTAGTTGTATGGTTAATACCAGATTGCAGCG
CGCAATTTCTAAAATTAAACCCACTTAGTAAACAGTCTGTTTACTGGGTAATCATGTA
TATGATAGTTTCTCCCAAACAAATACCCACGTTTATTGGGACAAAAGTTGTTAGGGA
AAATGGGGCCTCAGAGTTATGATTCAAGTCATAATTCTTTCCATTTATAATTTCACTCG

AGACTCTGTTAACTGATTCCTTGTGTGTTGTATCTTACTCCTCAGCTCACAATTACTTT
TAGTTATTCACCTTAACTGTATGAATAACAGTGGAGAAAAGGATTCTACCAGAATACT
CTAATTATGGTTTTGAGTCCCCTTTCCAGACTGAAGATTTTTCAGTCTTTTTGATCTGA
GGTGATTTTTCAGTCTTTTCGATCTGAGGTGACAGTCTCAAGCTCCTCAATTCACCCA
GTCTCTTGATACTTGTCCATTTAGGGCCACCAAAGCTACTTTGACTTCATACTAGAGA
GTCAATTAATGAGGCCATTCTCTGATGGACAGGTGAAGCAGGCAAGGTGACTATATT
TTGACTAAACGGTAGAAAACAGCCTGAGTGTTAACAGTGTAGCCTATAAAACCCAG
AGCTGCCCACCCTGATCTAAACTTCCAGGAACATAAGAAAGTAGGTCACATTTCAGT
AAAACCTGGCTTTGTGGATTGAGCATGGTCTGTCTCTTCCTGGTACTTCATTAGTCCC
CTAAGTGGGATTTGCTGAGCAAGACTGGCTCCGGTGCCCGTCAGTGGGCAGAGCGC
ACATCGCCCACAGTCCCCGAGAAGTTGGGGGGAGGGGTCGGCAATTGAACCGGTGC
CTAGAGAAGGTGGCGCGGGGTAAACTGGGAAAGTGATGTCGTGTACTGGCTCCGCC
TTTTTCCCGAGGGTGGGGGAGAACCGTATATAAGTGCAGTAGTCGCCGTGAACGTTC
TTTTTCGCAACGGGTTTGCCGCCAGAACACAGGTAAGTGCCGTGTGTGGTTCCCGC
GGGCCTGGCCTCTTTACGGGTTATGGCCCTTGCGTGCCTTGAATTACTTCCACCTGGC
TGCAGTACGTGATTCTTGATCCCGAGCTTCGGGTTGGAAGTGGGTGGGAGAGTTCG
AGGCCTTGCGCTTAAGGAGCCCCTTCGCCTCGTGCTTGAGTTGAGGCCTGGCCTGG
GCGCTGGGGCCGCCGCGTGCGAATCTGGTGGCACCTTCGCGCCTGTCTCGCTGCTTT
CGATAAGTCTCTAGCCATTTAAAATTTTTGATGACCTGCTGCGACGCTTTTTTTCTGG
CAAGATAGTCTTGTAAATGCGGGCCAAGATCTGCACACTGGTATTTCGGTTTTTGGG
GCCGCGGGCGGCGACGGGGCCCGTGCGTCCCAGCGCACATGTTCGGCGAGGCGGG
GCCTGCGAGCGCGGCCACCGAGAATCGGACGGGGGTAGTCTCAAGCTGGCCGGCCT
GCTCTGGTGCCTGGCCTCGCGCCGCCGTGTATCGCCCCGCCCTGGGCGGCAAGGCT
GGCCCGGTCGGCACCAGTTGCGTGAGCGGAAAGATGGCCGCTTCCCGGCCCTGCTG
CAGGGAGCTCAAAATGGAGGACGCGGCGCTCGGGAGAGCGGGCGGGTGAGTCACC
CACACAAAGGAAAAGGGCCTTTCCGTCCTCAGCCGTCGCTTCATGTGACTCCACGG
AGTACCGGGCGCCGTCCAGGCACCTCGATTAGTTCTCGAGCTTTTGGAGTACGTCGT
CTTTAGGTTGGGGGGAGGGGTTTTATGCGATGGAGTTTCCCCACACTGAGTGGGTGG
AGACTGAAGTTAGGCCAGCTTGGCACTTGATGTAATTCTCCTTGGAATTTGCCCTTTT
TGAGTTTGGATCTTGGTTCATTCTCAAGCCTCAGACAGTGGTTCAAAGTTTTTTTCTT
CCATTTCAGGTGTCGTGAACCATGTGGCCCCTGGTAGCGGCGCTGTTGCTGGGC
(SEQ ID NO:11).

Step 3: Following the method described in Example 1, the constructed cloning vector was transfected into competent cells, and positive colonies were selected and propagated. The plasmid was extracted and digested with NheI and HindIII restriction enzymes. The target DNA sequence was isolated and purified by gel electrophoresis, and then co-transfected with the H11-sgRNA-2 knockout vector into fibroblasts from TKO/CD55 transgenic pigs. GFP-positive cell populations were recovered by flow cytometry, and monoclonal cells were selected by limiting dilution. After culturing for about 10 days, the monoclonal cells were picked and identified at the DNA level using PCR technology, to obtain CD47 site-specific integration positive clones.

Step 4: Following the method described in Example 1, positive clone cells were injected into enucleated oocytes by somatic cloning technology for fusion. The reconstructed embryos were then transferred into the uterus of recipient sows for gestation.

Step 5: The recipient sows gave birth naturally to 7 healthy piglets, resulting in the construction of TKO/CD55/CD47 transgenic pigs.

The following steps involved identification at DNA level, identification at protein level, and protection efficacy verification for the born piglets:

### (1) Identification at DNA level

After the piglets were born, a small amount of ear tissue was taken, and DNA was extracted from the piglet ear samples using a DNA extraction kit according to the instructions. After DNA extraction, primers were designed based on the target CD47 transgenic DNA sequence for identification. There were three pairs of primers in total. The first pair of primers was used to identify the integrity of the promoter and CD47 cDNA region, and two remained pairs of primers, respectively near the left and right homologous arms, were used to identify the site-specific integration of CD47. After PCR amplification, the products were subjected to gel electrophoresis, to identify the site-specific integration of the left and right arms. All piglets showed site-specific integration of CD47 (Figure 9).

### (2) Identification at protein level

When piglets reached 1 month of age, 5 mL of whole pig blood was collected, centrifuged at 900 g for 5 min, and then washed twice with PBS. After separation of the RBCs, 10⁶ RBCs were taken out, to which was added 5 µL of FITC-labeled CD47 mouse anti-human antibody, and incubated at 4 °C for 30 min, followed by washing three times with PBS. The cells were re-suspended. The expression level of CD47 on the surface of RBCs was identified by flow cytometry. The expression level of CD47 was represented by the average fluorescence intensity. Wild-type pRBCs and hRBCs were used as controls.

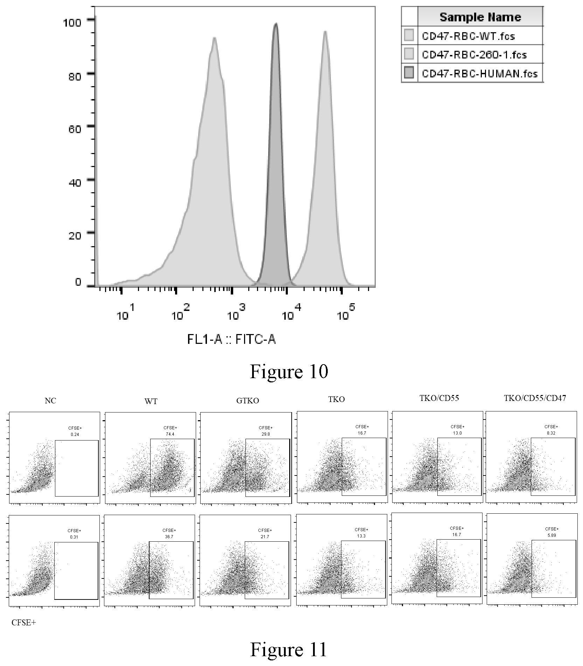

The results are shown in Figure 10. Wild-type pRBCs did not express human CD47 (orange), while human CD47 expression could be detected on the surface of pRBCs from TKO/CD55/CD47 transgenic pigs. Compared with hRBCs (red), the expression level of human CD47 on pRBCs from TKO/CD55/CD47 transgenic pigs (blue) was approximately 10 times higher.

### (3) Verification of protective effect

The method for verifying CD47 function by using monkey macrophages to phagocytize pRBCs was as follows:
Isolation of monkey macrophages: Fresh monkey lungs were collected, and the lower part of the larynx was clamped with double hemostatic forceps (or using a string for double ligation). The artery leading to the heart was ligated with a string, that was carefully operated to avoid damaging the integrity of the lungs. The foreign matter on the outside of the lungs was rinsed with a cleaning solution (1 × PBS + 10% RPMI-1640 + 3 × P/S). The lungs were placed in a tray, and transferred to a biosafety cabinet. The middle part between two hemostatic forceps was cut off. The tracheal opening was heated with an alcohol lamp, and washed with cleaning solution. A small amount of cleaning solution was poured into the lung through the trachea. The laryngeal tube was ligated with hemostatic forceps, the lung surface was gently massaged for 2-3 min, and the first lung wash fluid was collected in a sterile bottle. From time to time, PBS was used to rinse the lung surface to prevent it from drying out. Initially, a small amount of lavage fluid was administered, gradually increasing the amount thereafter. The second, third, and fourth rounds of lung lavage fluids were collected in the same manner (the number of washes depended on the turbidity of the fluid after washing). The obtained lung lavage fluid was filtered through a disposable cell sieve (70 mesh) into sterile bottles, and then aliquot into 50 mL sterile centrifuge tubes, followed by centrifuging at 1000 rpm for 4 min. The supernatant was discarded after centrifugation. The cell pellet was re-suspended in washing buffer, and centrifuged at 1000 rpm for 4 min. By using lysis buffer and following the instructions, RBCs were removed. Depending on the lysis condition, whether RBC lysis needed to be repeated was determined. After lysis, the cells were re-suspended in DMEM medium containing 10% serum.

pRBCs staining: After collecting 5 mL of pig whole blood, the blood was centrifuged at 900 g for 5 min, and then washed twice with PBS. After separation of the RBCs, 10⁶ RBCs were taken out, to which was added 1 mL of PBS solution containing 5 µM CFSE. The cells were incubated at 37 °C in the dark for 20 min. Subsequently, 5 mL of DMEM medium containing 1% serum was added, and then the cells were incubated at 37 °C in the dark for additional 5 min, followed by centrifuging at 1500 rpm for 5 min. The supernatant was removed, and the cells were re-suspended in DMEM medium containing 10% serum.

Phagocytosis: monkey macrophages and CFSE-labeled pRBCs were incubated together at a ratio of 1:1. After mixing the cells, they were placed in an incubator and incubated for 4 h. After incubation, RBC lysis buffer was used to remove the unphagocytosed pRBCs. Then, the cells were subjected to flow cytometry to detect the positive proportion of CFSE in the monkey macrophages, which represented the phagocytosed RBCs.

The results are shown in Figure 11. Compared with RBCs from TKO/CD55 pigs, the number of RBCs, that were from TKO/CD55/CD47 pig and phagocytosed by monkey macrophages, decreased by approximately 50%, indicating that the expression of CD47 significantly reduced the phagocytosis of pRBCs by monkey macrophages.

In summary, the present invention provided a method for genetically modifying pRBCs, which successfully expressed human protective proteins on pRBCs, enhanced the compatibility of pRBCs in the human immune system, reduced immune rejection, and lowered phagocytosis by macrophages. Relying on the fibroblasts derived from Triple-Knockout (TKO) pigs cultivated, with knockout of antigens α-gal (GGTA1 gene knockout), Sda (B4GalNT2 gene knockout), and Neu5Gc (CMAH gene knockout), the CRISPR-cas9 system was used to design sgRNA, achieving site-specific integration of exogenous DNA sequences at a safe harbor site within the pig genome. The EF1α promoter was employed to drive the expression of human-derived protective proteins, enabling the expression of such proteins on the surface of pRBCs. The transgenic strategy provided in the present invention enabled stable expression of exogenous genes on the surface of pRBCs, further enhancing the survival ability of pRBCs in the human body. It could be used as a reserve of RBCs from heterologous sources to alleviate the problem of insufficient blood reserves.

## Claims

1. A sgRNA, **characterized in that** its nucleotide sequence is set forth in SEQ ID NO:1, SEQ ID NO:2, SEQ ID NO:3, SEQ ID NO:4 or SEQ ID NO:5.

2. The sgRNA according to claim 1, **characterized in that** its nucleotide sequence is set forth in SEQ ID NO:3.

3. A sgRNA, **characterized in that** its nucleotide sequence is set forth in SEQ ID NO:7, SEQ ID NO:8, SEQ ID NO:9 or SEQ ID NO:10.

4. The sgRNA according to claim 1, **characterized in that** its nucleotide sequence is set forth in SEQ ID NO:8.

5. A method for constructing genetically engineered animals, **characterized in that** the method comprises the following steps:
(1) The sgRNA according to any one of claims 1-4 is connected with a vector to construct a knockout vector;
(2) Target DNA sequences expressing human protective proteins are constructed;
(3) Animal fibroblasts are co-transfected with knockout vectors and vectors containing target DNA sequences expressing human protective proteins, and positive clone cells with site-specific integration of human protective proteins are identified by sequencing;
(4) Using positive clone cells as nuclear donors and animal oocytes as recipients after enucleation, somatic cell nuclear transfer is performed to obtain reconstructed embryos;
(5) The reconstructed embryo is implanted into a female animal, the female animal is raised until it gives birth, and then the offspring is obtained.

6. The method according to claim 5, **characterized in that** in step (1), the vector is a pX458 vector.

7. The method according to claim 5, **characterized in that** in step (2), the human protective protein comprises one or both of human complement regulatory protein and human anti-cellular rejection protein; preferably, the human anti-cellular rejection protein is human anti-macrophage phagocytic protein or human anti-T cell activation protein; more preferably, the human complement regulatory protein is CD55, CD46, or CD59, the human anti-macrophage phagocytic protein is CD47, and the human anti-T cell activation protein is CTLA4.

8. The method according to claim 5, **characterized in that**, in step (2), the target DNA sequence expressing the human protective protein comprises a left homologous arm, an EF1α promoter, a CDS sequence of the human protective protein gene, a polyA sequence, a right homologous arm, and an HSV-TK sequence, which are sequentially connected; preferably, the target DNA sequence expressing the human protective protein is set forth in SEQ ID NO:6 or SEQ ID NO:11.

9. The method according to claim 5, **characterized in that** in step (3), the fibroblasts are fibroblasts with knockouts of heterologous antigens, and preferably fibroblasts with knockouts of α-gal, Sda, and Neu5Gc antigens.

10. The method according to any one of claims 5 to 9, **characterized in that** the animal is a pig.

11. The method according to any one of claims 5 to 10, **characterized in that** the method comprises the following steps:
(1) sgRNA is connected with the vector to construct knockout vector 1;
(2) Target DNA sequence 1 expressing human protective protein is constructed;
(3) Animal fibroblasts are co-transfected with knockout vector 1 and a vector containing the target DNA sequence 1 expressing human protective protein, and positive clone cells with site-specific integration of the human-derived protective protein are identified by sequencing;
(4) Using the positive clone cells obtained in step (3) as nuclear donors, and the enucleated animal oocytes as recipients, somatic cell nuclear transfer is performed to obtain reconstructed embryos;
(5) The reconstructed embryo obtained in step (4) is implanted into a female animal, the female animal is raised until it gives birth, and then the transgenic animal is obtained by taking the offspring;
(6) sgRNA is connected with the vector to construct knockout vector 2;
(7) Target DNA sequence 2 expressing human protective protein is constructed;
(8) The knockout vector 2 and the vector containing the target DNA sequence 2 expressing human protective protein are co-transfected into the transgenic animal fibroblasts in step (5), and identify the positive clone cells with site-specific integration of human protective protein by sequencing;
(9) Using the positive clone cells obtained in step (8) as nuclear donors, and the enucleated animal oocytes as recipients, the somatic cell nuclear transfer is performed to obtain reconstructed embryos;
(10) The reconstructed embryo obtained in step (9) is implanted into a female animal, the female animal is raised until it gives birth, and then the offspring is obtained;
wherein, sgRNA mentioned in step (1) is according to any one of claims 1-2, the target DNA sequence 1 for expressing the human protective protein mentioned in step (2) is set forth in SEQ ID NO:6, sgRNA mentioned in step (6) is according to any one of claims 3-4, and the target DNA sequence 2 for expressing the human-derived protective protein mentioned in step (7) is set forth in SEQ ID NO:11; alternatively, sgRNA mentioned in step (1) is according to any one of claims 3-4, the target DNA sequence 1 for expressing the human protective protein mentioned in step (2) is set forth in SEQ ID NO:11, sgRNA mentioned in step (6) is according to any one of claims 1-2, and the target DNA sequence 2 for expressing the human protective protein mentioned in step (7) is set forth in SEQ ID NO:6.

12. A genetically engineered animal constructed by the method according to any one of claims 5-11.

13. The use of genetically engineered animals according to claim 12 in xenogeneic blood transfusion.
